# EUROPÄISCHE PATENTANMELDUNG

(11) **EP 1 475 637 A1**
(43) Veröffentlichungstag der Anmeldung: **10.11.2004**
(21) Anmeldenummer: 03010495.4
(22) Anmeldetag: 09.05.2003
(51) Int. Cl.: G01N 33/15, G01N 21/35, A61B 5/103

(54) **Verfahren zur Prüfung der Hautverträglichkeit von Substanzen**

(71) Anmelder: Institut Dr. Schrader Creachem GmbH, 37603 Holzminden (DE)
(72) Erfinder: Schrader, Andreas, Dr., 37639 Bevern (DE); Rohr, Mathias, Dr., 34346 Hann. Münden (DE)
(74) Vertreter: Thalhammer, Wolfgang, Dr.

(57) **Zusammenfassung**

Beschrieben wird Verfahren zur Prüfung der Hautverträglichkeit einer Substanz, bei dem man die Substanz auf einen Bezirk der Haut einwirken lässt, und die Remission des Bezirks bei wenigstens einer Wellenlänge im Bereich von 1000 bis 1400 nm bestimmt. Das Verfahren eignet sich besonders zur Verträglichkeitsprüfung von Substanzen, die Bestandteil einer Farbmittel enthaltenden Zubereitung sind.

## Beschreibung

Die vorliegende Erfindung betrifft ein Verfahren zur Prüfung der Hautverträglichkeit einer Substanz oder eines Substanzgemisches.

Die Hautverträglichkeit natürlicher und/oder synthetischer Substanzen ist eine wichtige Voraussetzung für deren Verwendung als Wirk- und/oder Hilfsstoff in äußerlich anzuwendenden pharmazeutischen oder kosmetischen Zubereitungen. Die Hautverträglichkeit einer Substanz lässt sich z. B. prüfen, indem man die Substanz auf einen Bezirk der Haut, gegebenenfalls unter Okklusivbedingungen, einwirken lässt und nach einer Zeitspanne den Bezirk hinsichtlich des Auftretens entzündlicher Reaktionen untersucht. Entzündliche Reaktionen sind mit einer Rötung der Haut verbunden, die visuell oder durch Remissionsspektroskopie festgestellt werden kann. Dieses Verfahren versagt allerdings, wenn die zu untersuchende Substanz farbig ist und/oder die Haut anfärbt oder im Gemisch mit Substanzen vorliegt, die farbig sind und/oder die Haut anfärben, da die durch entzündliche Vorgänge hervorgerufene Farbveränderung der Haut durch die Eigenfarbe der Substanz oder die Anfärbung der Haut überdeckt oder verfälscht wird.

Es besteht daher ein Bedürfnis, insbesondere farbige und/oder färbende kosmetische Zubereitungen, wie Haarfärbemittel, Lippenstifte, Lidstriche, Wimperntuschen, Lidschatten, Grundierungen, Puder oder Make-ups, hinsichtlich ihrer Hautverträglichkeit zu prüfen.

M. Brandt, M. Rohr und A. Schrader berichten in IFSCC Magazine, Vol. 3, No. 1 (2000), 5-11, vom Auftreten einer Remissionsbande im NIR-Bereich, die mit der Induktion und dem Abklingen eines UV-induzierten Erythems korreliert.

Es wurde nun gefunden, dass die Chemikalien-induzierte Erythembildung der Haut mit dem Auftreten einer Remissionsbande im NIR-Bereich, genauer im Wellenlängenbereich von 1000 bis 1400 nm, einhergeht und die Detektion dieser Remissionsbande von Pigmenten oder anderen Farbmitteln nicht beeinträchtigt wird.

Die Erfindung betrifft daher ein Verfahren zur Prüfung der Hautverträglichkeit einer Substanz, bei dem man die Substanz auf einen Bezirk der Haut einwirken lässt, und die Remission des Bezirks bei wenigstens einer Wellenlänge im Bereich von 1000 bis 1400 nm, vorzugsweise 1100 bis 1200 nm, insbesondere etwa 1150 nm, bestimmt. Der Begriff "Substanz" soll vorliegend auch Substanzgemische umfassen.

Durch Vergleich des detektierten Signals mit Vergleichswerten der Remission des Hautbezirks, die vor der Einwirkung der Substanz bestimmt wurden, oder mit Vergleichswerten unbehandelter Hautbezirke kann man das Vorliegen und/oder den Grad entzündlicher Reaktionen feststellen und auf diese Weise Aussagen über die Hautverträglichkeit treffen.

Vorzugsweise wird die Substanz auf gesunde, d. h. nicht vorbehandelte oder vorgeschädigte, Haut aufgetragen.

Da die Detektion der Remissionsbande im NIR-Bereich von Pigmenten oder anderen Farbmitteln nicht wesentlich beeinträchtigt wird, eignet sich das erfindungsgemäße Verfahren besonders zur Prüfung der Hautverträglichkeit von Substanzen, die Bestandteil einer Farbmittel enthaltenden Zubereitung, üblicherweise einer pharmazeutischen oder kosmetischen Zubereitung, sind. Bei den Farbmitteln kann es sich um Pigmente, Farbstoffe, die üblicherweise zum Färben von Keratinfasern, z. B. der Haare, verwendet werden, wie Direktfarbstoffe, Oxidationsfarbstoffe oder Farbstoffvorläufer, Kuppler oder deren Gemische handeln. Pigmente sind z. B. Eisenoxide, Titandioxid, Tonerde, Glimmer oder organische Pigmente. Zu den üblichen Farbstoffvorläufern zählen p-Phenylendiamin-Derivate, wie p-Toluoldiamin, p-Phenylendiamin, N-2-Methoxyethyl-p-phenylendiamin, N,N-Bis(2-hydroxyethyl)-p-phenylendiamin, 2-Hydroxyethyl-p-phenylendiamin, 1-(2,5-Diaminophenyl)ethan-1,2-diol; p-Aminophenol-Derivate, wie p-Aminophenol, p-Methylaminophenol, 3-Methyl-p-aminophenol und 2-Methoxymethyl-p-aminophenol; ortho-Entwickler, wie o-Aminophenol, 1,2,4-Trihydroxybenzol, 2-Ethylamino-p-cresol, 5-Methyl-2-aminophenol, 6-Methyl-2-aminophenol und 2-Amino-5-acetaminophenol und heterocyclische Derivate,wie 2,4,5,6-Tetraaminopyrimidin und 4,5-Diamino-1-methylpyrazol. Zu den geeigneten Kupplern zählen zum Beispiel o-Aminophenol, Resorcin, 2-Chlorresorcin, 3-Aminophenol, 2,4-Diaminophenoxyethanol, 1-Naphthol, 2-Methyl-1-naphthol, 4-Chlorresorcin, 2-Methylresorcin, 5-Amino-2-methylphenol, 2-Amino-3-hydroxypyridin und deren Gemische.

Bei der pharmazeutischen oder kosmetischen Zubereitung kann es sich z. B. um ein Haarfärbemittel, einen Lippenstift, einen Lidstrich, eine Wimperntusche, einen Lidschatten, eine Grundierung, einen Puder oder ein Make-up handeln.

Zur praktischen Durchführung des erfindungsgemäßen Verfahrens verwendet man zweckmäßigerweise einen Bestrahlungskopf, in dem die Enden flexibler Lichtleiter für die remissionsanregende Strahlung und das von der Haut ausgesandte Remissionssignal angeordnet sind. Die Enden der Lichtleiter sind zweckmäßigerweise so im Bestrahlungskopf angeordnet, dass die von der Hautoberfläche direkt reflektierte Strahlung nicht in den Strahlengang für das remittierte Licht gelangt. Alternativ kann man aber auch ein Ende eines Y-förmigen Lichtleiters direkt auf die Hautoberfläche aufsetzen. Die anderen Enden der Lichtleiter sind mit einer Quelle für die Anregungsstrahlung, z. B. einer Xenonbogenlampe, bzw. dem NIR-Detektor für die Remissionsstrahlung verbunden. Als NIR-Detektor eignet sich beispielsweise eine InGaAs-Diode mit einer spektralen Empfindlichkeit im Bereich von 800 bis 1700 nm. Zwischen der Lichtquelle und dem Lichtleiter ist ein verstellbarer Monochromator angeordnet, der aus dem von der Lichtquelle gelieferten Licht eine spezifische Wellenlänge herausfiltert. Zweckmäßigerweise ist zur Verbesserung der Signalqualität vor dem Detektor ebenfalls ein Monochromator angeordnet, der mit dem Monochromator im Anregungsstrahlengang synchronisiert ist. Das detektierte Signal wird dann in Abhängigkeit von der anregenden Wellenlänge aufgezeichnet. Zur Kalibrierung wird geeigneterweise das Spektrum von Bariumsulfat aufgezeichnet, das als Referenz mit 100 % Remission dient.

Geeignete Messanordnungen sind z. B. in M. Brandt, M. Rohr und A. Schrader, IFSCC Magazine, Vol. 3, No. 1 (2000), 5-11, und der darin zitierten Literatur beschrieben.

Die Erfindung wird nun anhand der beigefügten Figuren und der folgenden Beispiele näher veranschaulicht.

Fig. 1 zeigt das Remissionsspektrum von Haut im Bereich von 1060 bis 1240 nm nach 5-, 10-, 20- und 30-minütiger Einwirkung von Ammoniaklösung. Dargestellt ist die Differenz der gemessenen Remission zur Remission vor dem Auftrag der Ammoniaklösung. Die Messwerte sind Mittelwerte von 10 Probanden.

Fig. 2 zeigt ein entsprechendes Remissionsspektrum, wobei die Haut vor dem Auftrag der Ammoniaklösung mit Tizian-Rot eingefärbt wurde.

Fig. 3 zeigt schematisch den Aufbau einer Messanordnung, die zur Remissionsmessung im Rahmen des erfindungsgemäßen Verfahrens geeignet ist.

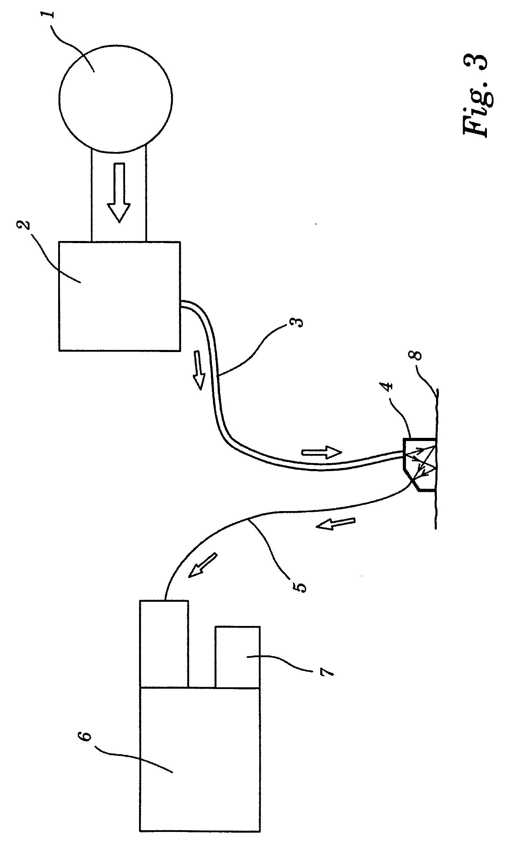

Mit Bezug auf Fig. 3 umfasst eine geeignete Anordnung zur Messung der Remission der Hautoberfläche einen Anregungsstrahlengang, der aus einer Lichtquelle 1, z. B. einer Xenon-Bogenlampe, einem Monochromator 2 und einem lichtzuführenden Lichtleiter 3 besteht, dessen Ende in einen Messkopf 4 mündet. Vom Messkopf 4 führt ein lichtabführender Lichtleiter 5 zum Monochromator 6, der einer Photodiode 7, z. B. einer InGaAs-Diode, vorgeschaltet ist. Der Messkopf 4 wird auf die Hautoberfläche 8 aufgesetzt.

### Beispiel

Auf ein etwa 4 x 4 cm großes Hautareal des Rückens eines Probanden wurden 30 µl 1 M Ammoniaklösung aufgetragen. Es wurden insgesamt 10 Probanden auf diese Weise behandelt. Nach 5, 10, 20 und 30 Minuten waren unterschiedlich stark ausgeprägte, visuell sichtbare Erytheme entstanden.

Mittels einer Messanordnung wie sie schematisch in Fig. 3 gezeigt ist wurden Remissionsspektren der behandelten Hautareale im Wellenlängenbereich von 1060 bis 1240 nm aufgezeichnet. Der verwendete Messkopf war etwa 4,3 x 3,0 x 3,5 cm groß. Die Bohrungen für den lichtzuführenden bzw. den lichtabführenden Lichtleiter waren so angeordnet, dass die Austrittsachsen der Lichtleiter einen Winkel von 45 ° einschlossen.

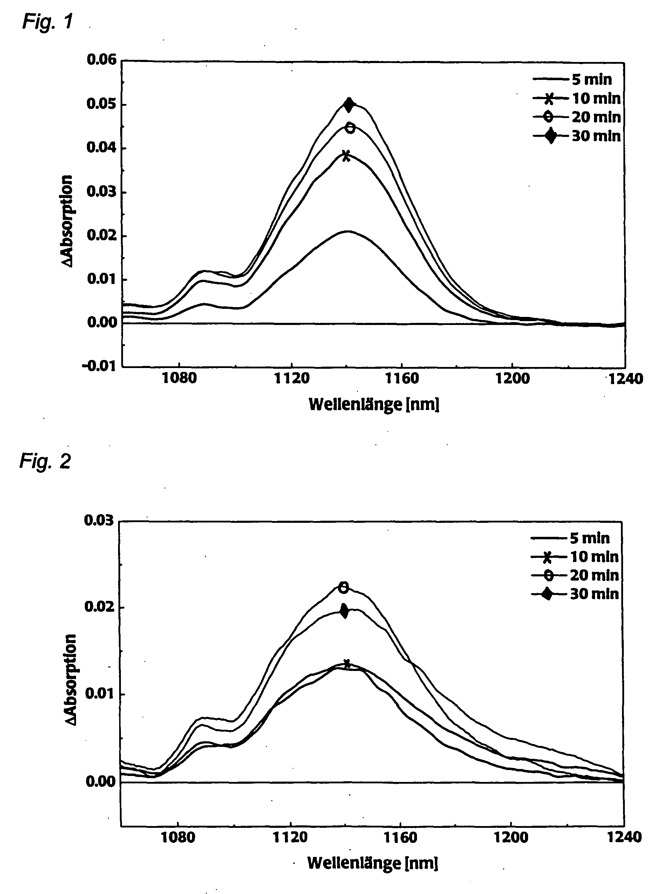

Die Mittelwerte der Remission (Differenz der gemessenen Remission zum Kontrollwert des Hautareals vor der Behandlung) sind in Fig. 1 in Abhängigkeit von der Wellenlänge dargestellt.

Der obige Versuch wurde wiederholt, wobei die Hautareale jedoch zuvor mit einer roten Permanenthaarfarbe (Tizian-Rot) eingefärbt wurden, so dass die auftretenden Erytheme visuell nicht mehr zu erkennen waren. Fig. 2 zeigt die dazugehörigen Remissionsspektren. Die Absorptionsbande bei etwa 1050 nm kann auch in diesem Versuch eindeutig identifiziert werden.

Die Versuche zeigen, dass die Identifizierung und Quantifizierung von Erythemen durch das erfindungsgemäße Verfahren auch ohne visuelle Kontrolle des Erythems möglich ist.

## Patentansprüche

1. Verfahren zur Prüfung der Hautverträglichkeit einer Substanz, bei dem man die Substanz auf einen Bezirk der Haut einwirken lässt, und die Remission des Bezirks bei wenigstens einer Wellenlänge im Bereich von 1000 bis 1400 nm bestimmt.

2. Verfahren nach Anspruch 1, bei dem man die Remission bei etwa 1150 nm bestimmt.

3. Verfahren nach Anspruch 1 oder 2, bei dem die Substanz Bestandteil einer Farbmittel enthaltenden Zubereitung ist.

4. Verfahren nach Anspruch 3, bei dem die Zubereitung eine pharmazeutische oder kosmetische Zubereitung ist.

5. Verfahren nach Anspruch 4, bei dem die Zubereitung unter einem Haarfärbemittel, einem Lippenstift, einem Lidstrich, einer Wimperntusche, einem Lidschatten, einer Grundierung, einem Puder und einem Makeup ausgewählt ist.
